# EUROPEAN PATENT APPLICATION

(11) **EP 4 803 003 A1**
(43) Date of publication of application: **09.09.2026**
(21) Application number: 26161290.7
(22) Date of filing: 27.02.2026
(51) Int. Cl.: A61B 5/327, A61B 5/00

(54) **ELECTROCARDIOGRAM SYSTEM**

(30) Priority: 03.03.2025 US 202563766006 P; 26.02.2026 US 202619551326
(71) Applicant: Pacesetter, Inc., Sylmar, CA 91342 (US)
(72) Inventor: Qu, Fujian, Sylmar, CA 91342 (US); Dawoud, Fady, Sylmar, CA 91342 (US); McSpadden, Luke, Sylmar, CA 91342 (US); Li, Wenwen, Sylmar, CA 91342 (US); Johnson, Eric, Sylmar, CA 91342 (US)
(74) Representative: Barker Brettell Sweden AB

(57) **Abstract**

An electrocardiogram (ECG) system (100) includes a computing device (102) configured to be communicatively connected to patch electrodes (104) that are affixed to skin of a patient. The computing device (102) includes one or more processors (118) configured to obtain electrical signals sensed by the patch electrodes (104) while the patch electrodes (104) are located at actual electrode locations. The processor(s) (118) are configured to generate initial ECG data based on the electrical signals according to assumed electrode locations on the patient that differ from the actual electrode locations. The processor(s) (118) are configured to convert the initial ECG data to converted ECG data by determining a relationship between the assumed electrode locations and the actual electrode locations; determining voltage values for reference vectors based on the initial ECG data and the relationship; and multiplying the voltage values for the reference vectors by a set of predetermined coefficients.

## Description

### TECHNICAL FIELD

Embodiments of the present disclosure relate generally to systems and methods that generate electrocardiograms using patch electrodes.

### BACKGROUND

Electrocardiogram (ECG or EKG) machines use patch electrodes to monitor electrical signals in the heart of a patient for diagnostic purposes. The patch electrodes are affixed to the skin of the patient. The electrical signals recorded by the ECG machines can be used to help diagnose irregular heart conditions. The ECG data can also be used to provide diagnostic feedback during various medical procedures, such as surgeries. In an example, ECG data may be collected during an implantable medical device (IMD) implant procedure. The ECG data may be used by a clinician to non-invasively analyze the effect of electrical stimulation provided by the IMD. During the implant procedure, the ECG data can assist the clinician with determining a selected pacing site for a lead of the IMD relative to the heart of the patient. Furthermore, ECG data may be collected during follow-up appointments to evaluate the continued effectiveness of pacing pulses and determine whether to modify pacing parameters of the IMD to improve the stimulation therapy provided by the IMD.

One example implant technique for pacing the heart is left bundle branch area pacing (LBBAP). In LBBAP, an implantable lead is routed into the heart and penetrates the interventricular septum between the left and right ventricles to reach the left bundle branch (LBB) of the conduction system of the heart. LBBAP is an efficient form of conduction system pacing (CSP), in which electrode(s) of the implantable lead deliver(s) electrical stimulation directly into the LBB. A clinician may utilize an ECG system to collect ECG data to assist with selecting a pacing site for the implantable lead in the LBB during the implant procedure and/or modifying pacing parameters of the IMD after lead implant in the LBB. Current LBBAP techniques employ both V1 and V6 ECG lead tracings to continuously assess ECG morphology changes and deflection peak time delay. The morphology changes and deflection peak time delay can be used by a clinician or automated device to select a pacing site for successful LBBAP. The morphology changes and deflection peak time delay can also be used to program pacing parameters of the IMD (e.g., control the electrical stimulation emitted by the implantable lead into the LBB). A system that can concurrently provide multiple ECG lead outputs, including V1 and V6, would improve the efficiency and effectiveness of the IMD implant procedure for LBBAP, as well as enable non-invasive follow-up evaluation for LBBAP implants.

The conventional ECG machine collects electrical signals from at least ten surface electrodes to generate 12-lead ECG data. The 12-lead ECG data includes the V1 and V6 lead tracings that can assist with LBBAP implants. However, the conventional ECG system is relatively large and generally used at one or more fixed locations in the hospital. The conventional ECG system may not be portable. Furthermore, many clinician offices and/or healthcare facilities may not have access to a conventional ECG system. Accordingly, the conventional 12-lead ECG system may not be available to assist with some LBBAP implant and/or follow-up evaluation procedures. The conventional 12-lead ECG system also may be relatively time-consuming and complex to use, as the clinician needs to affix and electrically connect at least ten patch electrodes to the patient. There is a need for a more portable and efficient ECG system that can provide the standard 12-lead ECG data, including concurrent V1 and V6 lead data, for assisting with LBBAP procedures.

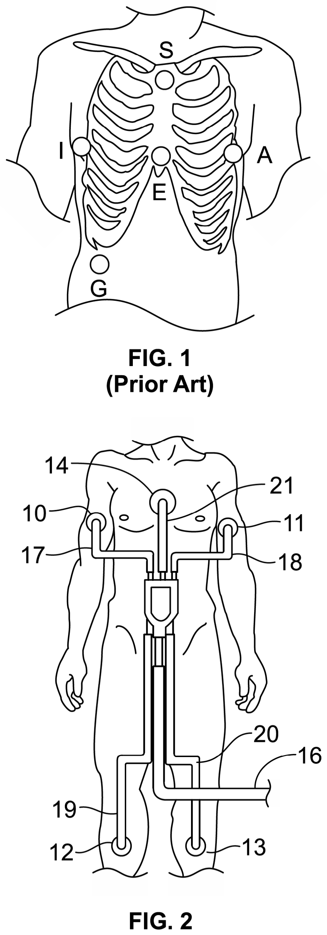

A lead system called EASI uses a modified setup to derive the standard 12-lead ECG signals using a reduced number of electrodes on the patient, referred to herein as a reduced electrode configuration. In an example, the reduced electrode configuration may utilize only five patch electrodes. The EASI lead system was proposed in the document Dower GE, Yakush A, Nazzal SB, et al: Deriving the 12-lead electrocardiogram from four (EASI) electrodes, J Electrocardiol 21:182, 1988. The EASI lead system uses electrode locations that are on the patient's torso. Figure 1 illustrates the EASI electrode locations relative to a patient torso and rib cage. For example, electrode locations referred to as "I," "E," and "A" are at right, center, and left positions, respectively, of the chest approximately in a horizontal line that crosses the lower sternum area of the rib cage. The electrode location referred to as "S" is centrally located at the upper sternum area (e.g., manubrium). A reference or ground ("G") electrode location may be below the rib cage, or at least below the other four electrode locations. By being on the torso, the EASI electrode locations may be relatively easy-to-locate, stable anatomical sites that leave the precordium free for other diagnostic and/or invasive procedures. The EASI lead system derives the 12-lead ECG data from three bipolar reference vectors between the patch electrodes located at the EASI electrode locations using a set of pre-determined coefficients. The reference vectors are referred to as "AS," "ES," and "AI." For example, the EASI lead system may measure the voltages (e.g., electrical potentials) at the three reference vectors AS, ES, and AI, and then multiplies the voltage values by the pre-determined coefficients in the set to generate 12-lead ECG data. The coefficients in the set may be derived from historical data collected from a large sample of patients. Thus, the EASI lead system beneficially generates 12-lead ECG data using a reduced number of patch electrodes. Devices that implement the EASI lead system can be smaller, more portable, and less complex than conventional ECG machines.

However, some ECG devices with reduced electrode configurations are designed for placing the electrodes at different locations from the EASI electrode locations, and cannot be directly adapted to achieve the derived 12-lead ECG data according to the EASI technique described above. For example, some ECG devices are designed to generate ECG data based on affixing five patch electrodes to limbs and chest of the patient. Figure 2 shows example electrode locations for a system that generates ECG data using five electrodes 10-14. The ECG data generated by the system in Figure 2 may be 7-lead ECG data. The first electrode 10 is affixed to the right arm of the patient. The second electrode 11 is affixed to the left arm. The third and fourth electrodes 12, 13 are affixed to the right and left legs, respectively. The fifth electrode 14 is affixed to the chest of the patient, near the mid or upper portion of the sternum. The system in Figure 2 includes an electrical cable 16 that has five electrical wires 17-21. Each of the five wires 17-21 is electrically connected to a different one of the five patch electrodes 10-14. The electrical cable 16 conveys electrical signals from the five patch electrodes 17-21 to an ECG device which analyzes the signals and generates the ECG data. The ECG device may be designed to compute and output the ECG data using pre-determined formula based on the four limb electrode locations and the chest electrode location. When the five patch electrodes 10-14 are repositioned to the EASI locations, the ECG data calculated using pre-determined formula are not directly applicable for the EASI reference vectors AS, ES, and AI which measure the potential difference from the E to S locations, the A to S locations, and the A to I locations.

There is a need for such ECG system that can convert the initial ECG data output, generated via a reduced electrode configuration, to EASI ECG vectors to then derive the 12-lead ECG data. By deriving 12-lead ECG data using the reduced electrode configuration (e.g., fewer than the conventional 10 patch electrodes), the ECG system could assist with LBBAP implant and follow-up evaluation applications, among other procedures. There is a need for updating an ECG system that is designed to output 7-lead ECG data, for example, so that the ECG system can generate 12-lead ECG data without requiring additional hardware or modified hardware.

### SUMMARY

In accordance with embodiments herein, an electrocardiogram (ECG) system includes a computing device configured to be communicatively connected to patch electrodes that are affixed to skin of a patient. The computing device includes one or more processors configured to obtain electrical signals sensed by the patch electrodes while the patch electrodes are located on the patient at actual electrode locations. The one or more processors are configured to generate initial ECG data based on the electrical signals from the patch electrodes according to assumed electrode locations on the patient that differ from the actual electrode locations, and convert the initial ECG data to converted ECG data. The one or more processors convert the initial ECG data by determining a relationship between the assumed electrode locations and the actual electrode locations, and determining voltage values for reference vectors based on the initial ECG data and the relationship that is determined. The one or more processors generate the converted ECG data by multiplying the voltage values for the reference vectors by a set of predetermined coefficients.

In accordance with embodiments herein, a method includes obtaining, via one or more processors of a computing device, electrical signals sensed by patch electrodes. The patch electrodes sense the electrical signals while affixed to skin of a patient at actual electrode locations. The method includes generating initial ECG data based on the electrical signals received from the patch electrodes according to assumed electrode locations on the patient that differ from the actual electrode locations. The method includes converting the initial ECG data to converted ECG data. The initial ECG data is converted by determining a relationship between the assumed electrode locations and the actual electrode locations, and determining voltage values for reference vectors based on the initial ECG data and the relationship that is determined. The converted ECG data is generated by multiplying the voltage values for the reference vectors by a set of predetermined coefficients.

### BRIEF DESCRIPTION OF THE DRAWINGS

Figure 1 illustrates EASI-specific electrode locations relative to a patient torso and rib cage.
Figure 2 shows example electrode locations for a system that generates 7-lead ECG data using five electrodes.
Figure 3 illustrates an ECG system according to an embodiment.
Figure 4 illustrates patch electrodes of the ECG system poised for attachment to a patient at the EASI-specific electrode locations.
Figure 5 is a flow chart of a method for generating 12-lead ECG data using a reduced number of electrodes according to an embodiment.

### DETAILED DESCRIPTION

It will be readily understood that the components of the embodiments as generally described and illustrated in the figures herein may be arranged and designed in a wide variety of different configurations in addition to the described example embodiments. Thus, the following more detailed description of the example embodiments, as represented in the figures, is not intended to limit the scope of the embodiments, as claimed, but is merely representative of example embodiments.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, etc. In other instances, well-known structures, materials, or operations are not shown or described in detail to avoid obfuscation. The following description is intended only by way of example, and simply illustrates certain example embodiments.

The methods described herein may employ structures or aspects of various embodiments (e.g., systems and/or implantable leads and/or IMDs) discussed herein. In various embodiments, certain operations may be omitted or added, certain operations may be combined, certain operations may be performed simultaneously, certain operations may be performed concurrently, certain operations may be split into multiple operations, certain operations may be performed in a different order, or certain operations or series of operations may be re-performed in an iterative fashion. It should be noted that, other methods may be used, in accordance with an embodiment herein. Further, wherein indicated, the methods may be fully or partially implemented by one or more processors of one or more IMDs, devices, or systems. While the operations of some methods may be described as performed by the processor(s) of one device, additionally, some or all of such operations may be performed by the processor(s) of another device described herein.

Embodiments may be implemented in connection with one or more implantable medical devices (IMDs). Non-limiting examples of IMDs include neurostimulator devices, implantable leadless monitoring and/or therapy devices, and/or alternative implantable medical devices. For example, the IMD may represent a cardiac monitoring device, pacemaker, cardioverter, cardiac rhythm management device, defibrillator, neurostimulator, leadless monitoring device, leadless pacemaker and the like. For example, the IMD may include one or more structural and/or functional aspects of the device(s) described in U.S. Patent 9,333,351 "Neurostimulation Method And System To Treat Apnea" and U.S. Patent 9,044,610 "System And Methods For Providing A Distributed Virtual Stimulation Cathode For Use With An Implantable Neurostimulation System".

Embodiments set forth herein describe an ECG system that generates initial ECG data, using a reduced electrode configuration, and converts the initial ECG data to converted, output ECG data. The ECG system includes a computing device that is communicatively connected to patch electrodes. In an example, the computing device may be electrically connected to the patch electrodes via multiple wires. The patch electrodes are affixed to the skin of a patient at so called actual electrode locations, and sense electrical signals that are conveyed via the wires to the computing device. The computing device is referred to herein as a "programmer device," but may be any type of computing device able to perform the operations described herein attributed to the programmer device. The initial ECG data preferably has fewer leads than the converted ECG data. The ECG system described herein uses the initial ECG data, as well as a relationship between the actual patch electrode locations and assumed patch electrode locations, to derive data for additional leads, yielding the converted ECG data. In one or more embodiments, the initial ECG data is 7-lead ECG data, and the converted ECG data is 12-lead ECG data. Although the examples below refer to 7-lead ECG data, the initial ECG data is not limited to having seven leads. Furthermore, the converted ECG data is not limited to having twelve leads, although examples below refer to the converted ECG data as 12-lead ECG data. As described above, 12-lead ECG data is conventionally calculated using ten or so patch electrodes. Embodiments of the ECG system described herein use fewer than ten patch electrodes to generate the initial ECG data. Several examples described herein utilize only five patch electrodes to generate the 7-lead ECG data that represents the initial ECG data. The ECG system is therefore able to derive 12-lead ECG data based on electrical signals sensed by fewer than ten electrodes, such as only five electrodes.

In an example embodiment, the computing device of the ECG system may use the 7-lead ECG leads (or tracings) to calculate bipolar potentials between electrodes, even when the electrodes are not positioned at originally designated locations, such as on the limbs and chest of the patient. The computing device may determine the potential differences (e.g., in volts) of specific bipolar reference vectors without directly measuring the potential differences. The computing device may use the available 7-lead ECG channels, which are typically the output, as the input to derived mathematical transfer functions to calculate the voltages along the reference vectors. The mathematical transfer functions may be selected based on a relationship between the actual locations of the patch electrodes on the patient (e.g., the electrode configuration) and expected or assumed electrode locations on the patient. The reference vectors are vectors between pairs of patch electrodes at the actual electrode locations on the patient. In an example, the reference vectors are EASI vectors AS, ES, and AI. Once the voltages of the reference vectors (e.g., EASI reference vectors) are determined based on the initial ECG data and the relationship between the actual and assumed electron locations, the computing device may calculate the 12-lead ECG data based on the reference voltages and a set of pre-determined coefficients. The set of pre-determined coefficients may be accessible from a memory storage device, communication with a remote device, or the like. For example, the voltages of the EASI reference vectors may be multiplied by corresponding coefficients in the set to generate the 12-lead ECG data.

The ECG system described herein has several beneficial technical effects. For example, the ECG system described herein may be more portable, less complex, and less expensive than traditional 12-lead ECG systems, due in part to using a reduced number of electrodes. As a result, the ECG system described herein may be more readily available and/or accessible in clinician offices and surgical facilities than the traditional 12-lead ECG systems.

In another example, the ECG system described herein generates 12-lead ECG data that can assist with LBBAP implant procedures and/or follow-up pacing effectiveness evaluation procedures. For example, the ECG system may output concurrent ECG data along multiple leads (e.g., V1 and V6). The concurrent multi-lead ECG data allows for continuously assessing ECG morphology changes and deflection peak time delay. The ECG system may display the 12-lead ECG data on a display device. This ECG data allows a clinician and/or the ECG system itself to analyze a pacing site and/or pacing parameters, and adjust the lead placement and/or pacing parameters to improve the LBBAP therapy (relative to not adjusting the lead placement and/or pacing parameters).

At least one technical effect is that the ECG system described herein may achieve generating 12-lead ECG data without modifying the hardware already used in at least some systems that generate 7-lead ECG data. For example, the ECG system described herein may be accomplished via a software update or add-on. As a result, the ECG system described herein may have the hardware of a 7-lead ECG system but provides the 12-lead ECG output. Converting the 7-lead ECG data to 12-lead ECG data enables the ECG system to assist with procedures, such as LBBAP procedures, that require more than the information supplied in the 7-lead ECG data.

The ECG system according to one or more embodiments uses EASI electrode locations and EASI reference vectors, even though the ECG system is not necessarily set-up or configured to generate ECG data in accordance with the EASI technique. For example, the ECG system according to one or more embodiments may be set-up or configured for an electrode configuration as shown in Figure 2, which includes one or more patch electrodes located along limbs of the patient. The EASI electrode locations and EASI reference vectors enables the ECG system to use a derived set of EASI coefficients for generating the 12-lead ECG data based on the calculated voltage values of the reference vectors. The inventive subject matter described herein is, however, not be limited to EASI electrode placement and reference vectors (e.g., AS, ES, and AI). For example, the ECG system may calculate bipolar ECG vectors from device-recorded ECG signals regardless of electrode placement. The transfer formulas and/or coefficients may change based on rearranging and/or repositioning the electrodes on the patient to deviate from the EASI locations.

The generation of the 12-lead ECG data based on the 7-lead (e.g., EASI) ECG data may be an approximation or close estimation, rather than an exact derivation or transformation. For example, not all resulting 12-lead vectors may perfectly resemble the standard 12-lead ECG. In an embodiment, the system uses a pre-specified set of coefficients (e.g., EASI coefficients) to approximate 12-lead ECG data for the patient. The same set of coefficients can be utilized for all patients. In another example, the system described herein may allow a user to customize the coefficients for individual patients. In a clinic, the user can measure 12-lead data using a conventional ECG 12-lead system. The software of the system may then calibrate and/or adjust the EASI coefficients, based on the user-specific 12-lead data, to determine an approximation for the specific patient, particularly on the V1 and V6 leads. The system may store the modified EASI coefficients for the patient in a storage device, such as a programmer device, for availability to retrieve during a subsequent LBBAP procedure.

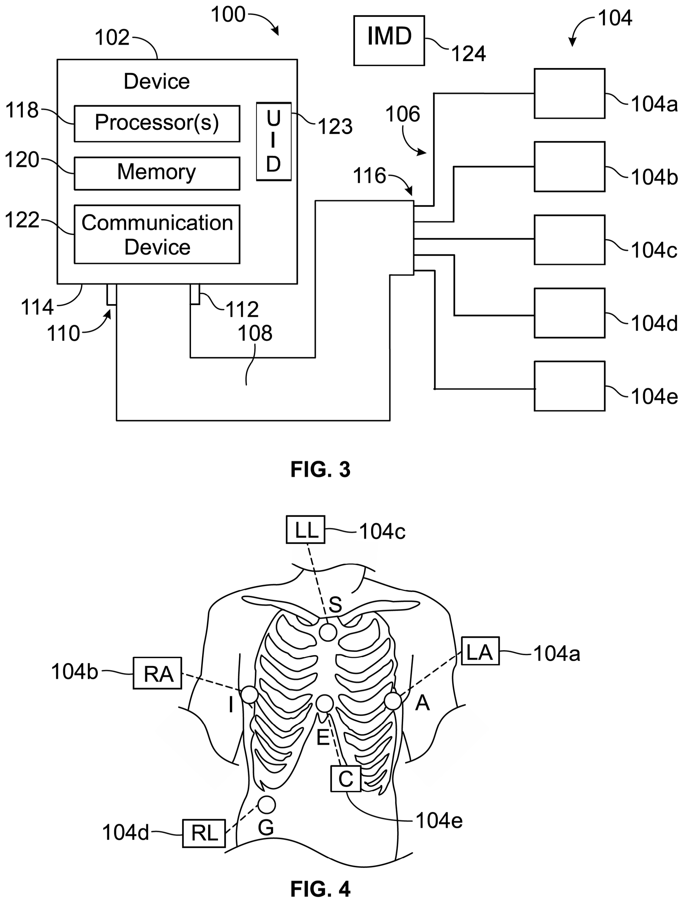

Figure 3 illustrates an ECG system 100 according to an embodiment. The ECG system 100 includes a computing device 102. The computing device 102 may be a programmer device 102 that communicates with an IMD 124. The programmer device 102 may be used by a clinician to select and/or modify electrical stimulation therapy parameters to be implemented by the IMD 124 within a patient. The clinician is a person that may be a medical physician or other medical technician trained to interpret ECG data. The ECG system 100 may be used to generate converted ECG data that shows electrical properties of the patient. The converted ECG data may be used by the clinician to monitor cardiac activity of the patient's heart. Based at least in part on the feedback provided in the form of the converted ECG data, the clinician may select and/or modify the stimulation therapy parameters implemented by the IMD 124. The programmer device 102 may then communicate the updated parameters to the IMD 124. Various examples described herein refer to the computing device 102 of the ECG system 100 as programmer device 102, but the device 102 may not be an IMD programmer device in other embodiments.

The programmer device 102 is communicatively connected to multiple patch electrodes 104. In an example, the programmer device 102 is electrically connected to the patch electrodes 104 via multiple electrical wires 106. Each electrical wire 106 may be electrically connected to a different corresponding one of the patch electrodes 104. The electrical wires 106 may be individually insulated. In another example, the patch electrodes 104 may be wirelessly connected to the programmer device 102 via an inductive circuit or the wireless transmission of electrical signals. The patch electrodes 104 are designed to be affixed to the external surface of the skin of a patient at so-called actual electrode location. The patch electrodes 104 may be secured in place on the patient's skin via an adhesive. The electrical wires 106 have sufficient length to enable the patch electrodes 104 to be affixed to the patient at different locations on the patient's body. The patch electrodes 104 may sense electrical signals within the patient's body. The electrical signals are conveyed via the electrical wires 106 to the programmer device 102 for analysis. In an example, the ECG system 100 includes five patch electrodes 104a, 104b, 104c, 104d, 104e and five corresponding electrical wires 106. The ECG system 100 may have more or less than five electrodes in other embodiments.

The electrical wires 106 may be part of an electrical cable 108 that electrically connects to the programmer device 102. For example, the electrical cable 108 may include an outer jacket or sheath that collectively surrounds the electrical wires 106 along at least some of the distance from the programmer device 102 to the electrodes 104. A proximal end 110 of the electrical cable 108 may have a connector that removably connects to a port 112 of the programmer device 102. The port 112 may be located along a side of a housing 114 of the programmer device 102. The electrical wires 106 may project from a distal end 116 of the electrical cable 108. In an alternative embodiment, the electrical wires 106 may not be collected into a single electrical cable 108. For example, the electrical wires 106 may independently connect to the programmer device 102.

The programmer device 102 includes one or more processors 118 that perform the operations of the programmer device 102 described herein. The one or more processors 118 represent hardware circuitry, such as one or more microprocessors, integrated circuits, microcontrollers, field programmable gate arrays, etc.). The processor(s) 118 may operate by executing program instructions in the form of software. The programmer device 102 may include at least one tangible and non-transitory computer-readable storage medium (e.g., data storage device), referred to herein as memory 120. The memory 120 may store the program instructions (e.g., software) that are executed by the one or more processors 118 to perform the operations of the programmer device 102 described herein.

In an example, the one or more processors 118 may be organized into multiple modules or circuits that communicate with each other to perform the operations. For example, a first or primary module may receive electrical signals sensed by the patch electrodes 104 and may generate 7-lead (e.g., initial) ECG data. A secondary module may receive the 7-lead ECG data that is output by the primary module. The secondary module may convert the 7-lead ECG data to 12-lead (e.g., converted) ECG data. The secondary module may perform the conversion process by determining voltage values for reference bipolar vectors based on the relationship between the assumed and actual electrode locations and the 7-lead ECG data that is output by the primary module. After determining the voltage values of the reference vectors, the secondary module may multiply the voltage values of the reference vectors by a set of predetermined coefficients to generate the 12-lead ECG data. In this example embodiment, the operations of the secondary module may be accomplished by a software update or add-in. For example, the operations of the primary module may be dictated by legacy software, and new software that is added to the programmer device 102 may control the operations of the secondary module. Although two different modules are described in this example, the one or more processors 118 may be arranged in a single circuit or module to perform all of these operations in another example.

The programmer device 102 may include a communication device 122 that is used for wirelessly communicating with an IMD 124 configured to be implanted within the patient. The communication device 122 may also enable the programmer device 102 to communicate with other external devices. The communication device 122 may include or represent circuitry for wirelessly communicating electrical signals. For example, the communication device 122 can represent transceiving circuitry, at least one antenna, and associated circuitry. The transceiving circuitry may include a transceiver or a separate transmitter and receiver. The communication device may communicate RF electrical signals, inductive signals, or conductive electrical signals as illustrative, but non-limiting, examples. The electrical signals can represent data packets that form messages in the aggregate. The data in the messages may indicate selected pacing parameters to be implemented by the IMD 124 that receives the messages. Some electrical signals communicated by the communication device 122 may be control signals. In addition to sending messages, the communication device 122 may receive messages that are forwarded to the processor(s) 118 for analysis of the contents of the received messages.

The programmer device 102 may include at least one user input device 123 (abbreviated "UID" in Figure 3) configured to allow a user/operator of the programmer device 102 to provide user selections. For example, the user input device 123 may include or represent a keyboard, a physical button, a touch screen, a touch pad, and/or the like. In an example, the programmer device 102 includes or is communicatively connected to a display device. The display device displays a graphical user interface (GUI) specific to the ECG system 100. The user may manipulate the user input device 123 to make selections on the GUI and/or input data into windows on the GUI.

In an example, the ECG system 100 is able to switch between a data conversion mode of operation and a standard mode of operation. In the data conversion mode, the ECG system 100 first generates initial ECG data and then converts the initial ECG data to converted ECG data. For example, the ECG system 100 may first generate 7-lead ECG data and then converts the 7-lead ECG data to 12-lead ECG data, without using any additional patch electrodes. The converted ECG data is output by the ECG system 100 in the data conversion mode. In the standard mode, on the other hand, the ECG system 100 may generate the initial ECG data and then output that initial ECG data without converting the initial ECG data. For example, the ECG system 100 may output 7-lead ECG data in the standard mode. The ECG system 100 does not convert 7-lead ECG data to 12-lead ECG data in the standard mode. In an example, the user may manipulate the user input device 123 to toggle between the data conversion mode and the standard mode, as desired. For example, the user input device 123 may generate a user selection signal, based on a touch input provided by the user, which is conveyed to the one or more processors 118 of the ECG system 100. Upon receipt of the user selection signal, the one or more processors 118 may switch from the standard mode to the data conversion mode, or vice-versa. In an example, the GUI may present a virtual button for toggling between the two modes.

In an example, the ECG system 100 may use the GUI to set up a feedback look for clinicians to report feedback, such as issues experienced while using the ECG system 100. The processor(s) 118 may generate a prompt displayed on the GUI that requests the user clinician to input feedback using the user input device 123.

A clinician may set up the ECG system 100, for generating the ECG data, by affixing the patch electrodes 104 to the skin of the patient at multiple locations. In an example, the clinician affixes the patch electrodes 104 to the different locations on the torso of the patient. The locations of the patch electrodes 104 on the torso may be EASI-specific locations. The EASI-specific locations include a left side chest location (shown as "A" in Figure 1), a right side chest location (shown as "I" in Figure 1), an upper sternum location (shown as "S" in Figure 1), a lower sternum location (shown as "E" in Figure 1), and a ground location (shown as "G" in Figure 1). The left side chest location may be along the left midaxillary line of the patient. The right side chest location may be along the right midaxillary line of the patient. The upper sternum location may be along the patient's manubrium. The lower sternum location may be along the patient's xiphoid process. The ground location may be below the other electrode locations. For example, the ground location may be below the sixth rib of the patient's rib cage. The clinician may affix the patch electrodes 104 to the skin using medical grade adhesive.

Figure 4 illustrates the patch electrodes 104 poised for attachment to the patient at the EASI-specific locations (A, E, S, I, and G). In the illustrated electrode configuration, the first electrode 104a is affixed at the left side chest (A) location. The second electrode 104b is affixed at the right side chest (I) location. The third electrode 104c is affixed at the upper sternum (S) location. The fourth electrode 104d is affixed at the ground (G) location. The fifth electrode 104e is affixed at the lower sternum (E) location.

The electrode 104a-e are labeled LA, RA, LL, RL, and C due to the example electrode locations shown in Figure 2 for a conventional system that outputs 7-lead ECG data only. When used in the conventional system, the first electrode 104a is expected to be affixed to the patient's left arm (LA), the second electrode 104b is expected to be affixed to the patient's right arm (RA), the third electrode 104c is expected to be affixed to the patient's left leg (LL), the fourth electrode 104d is expected to be affixed to the patient's right leg (RL), and the fifth electrode 104e is expected to be affixed to the patient's chest (C).

The set-up process also includes establishing electrical connections between the programmer device 102 and the patch electrodes 104. The clinician may connect distal ends of the wires 106 to the patch electrodes 104 and proximal ends to the programmer device 102 to provide conductive signal pathways along the wires 106. In an example, each of the first electrical wires 106 may be connected to a different corresponding patch electrode 104. When the wires 106 are part of an electrical cable 108, as shown in Figure 3, the clinician may plug the proximal end 110 of the cable 108 into the port 112 of the programmer device 102.

When activated, the programmer device 102 may receive electrical signals sensed by the patch electrodes 104 while the patch electrodes 104 are located on the patient at the actual electrode locations. The electrical signals may be conveyed to the programmer device 102 via the wires 106. The ECG system 100 may be activated to non-invasively monitor a condition of the patient. The one or more processors 118 receive and analyze the electrical signals to first generate initial ECG data (e.g., 7-lead ECG data). The 7-lead ECG data in this case is intermediate data, not a final product that is output by the ECG system 100. The 7-lead ECG data may include data for six limb leads (e.g., vectors) associated with the arms and legs of the patient and one precordial lead associated with the chest of the patient, such as exemplified in Figure 2. For example, the leads of the 7-lead ECG data may include *I, II, III, aVR, aVL, aVF,* and *V*, where *V* is associated with the chest and the other six leads are limb leads. This 7-lead ECG data at these actual patch locations is inaccurate because the calculations used to generate the seven leads are prefaced on the patch electrodes 104 being affixed at assumed electrode locations that differ from the actual electrode locations of the patch electrodes 104. For example, the first electrode 104a is expected (or assumed) to be secured to the left arm, but is actually at the left side of the chest. In another example, the third electrode 104c is expected to be on the left leg, but is actually along the upper sternum area of the chest. The processor(s) 118 use this initial ECG data to derive converted ECG data for additional leads without requiring hardware updates on the ECG system 100. For example, the ECG system 100 may convert 7-lead ECG data to 12-lead ECG data without using more than five patch electrodes 104 affixed to the patient.

The processor(s) 118 convert the 7-lead ECG data to 12-lead ECG data by a series of steps. The processor(s) 118 determine a relationship between assumed electrode locations of the patch electrodes 104, such as shown in Figure 2, and actual electrode locations of the patch electrodes 104 on the patient, such as shown in Figure 4. The actual electrode locations refer to the electrode configuration or arrangement of electrodes 104 along the patient's torso. The relationship refers to a correspondence between an expected location of each patch electrode 104 and the actual location of that patch electrode 104. For example, one aspect of the relationship may be that the right leg (RL) electrode 104d is affixed to the patient at a ground (G) location along the patient's torso below the rib cage, rather than the assumed location on the patient's right leg.

That relationship may be used to select mathematical transfer functions. In an embodiment, the actual electrode locations are EASI-specific locations of the patch electrodes 104 currently affixed to the patient. The EASI-specific locations may all be on the patient's torso. One or more of the assumed electrode locations may be on one or more limbs of the patient. For example, the assumed electrode locations may include a left arm (LA) of the patient, a left leg (LL) of the patient, a right arm (RA) of the patient, a right leg (RL) of the patient, and a chest (C) of the patient. To be clear, the patch electrodes 104 of the ECG system 100 sense electrical signals from the patient while affixed to the patient at the actual electrode locations, such as the EASI-specific locations, and not the assumed electrode locations.

The processor(s) 118 determine voltage values for reference vectors based on values of the initial (e.g., 7-lead) ECG data and the relationship between the assumed electrode locations and the actual electrode locations. The processor(s) 118 may determine the voltage values for the reference vectors by inputting the values of the initial ECG data into mathematical transfer functions that output the voltage values for the reference vectors. The processor(s) 118 select or derive the mathematical transfer functions based on the relationship between the assumed electrode locations and the actual electrode locations. The reference vectors are reference vectors between pairs of patch electrodes at the actual electrode locations. In an embodiment, the reference vectors are EASI reference vectors. The EASI reference vectors may include an AS vector, an ES vector, and an AI vector.

Then, the processor(s) 118 multiply the voltage values for the reference vectors by a set of predetermined coefficients to generate the converted (e.g., 12-lead) ECG data. The converted ECG data may be output, such as to a display device for visual presentation to the clinician.

As shown in Figures 1 and 4, the AS vector has a diagonal orientation from the left side chest (A) location to the upper sternum (S) location. In the illustrated electrode configuration in Figure 4, the AS vector is the potential difference from the first patch electrode 104a (the LA electrode) to the third patch electrode 104c (LL electrode). The AI vector has a horizontal orientation from the left side chest (A) location to the right side chest (I) location. As shown in Figure 4, the AI vector is the potential difference from the first patch electrode 104a (the LA electrode) to the second patch electrode 104b (RA electrode). The ES vector has a vertical orientation from the lower sternum (E) location to the upper sternum (S) location. In the illustrated electrode configuration, the ES vector is the potential difference from the fourth electrode 104e (C electrode) to the third electrode 104c (LL electrode).

The one or more processors 118 may determine the voltage values of the EASI reference vectors by inputting values of at least some leads of the 7-lead ECG data into the selected mathematical transfer functions. The mathematical transfer functions output the voltage values of the EASI reference vectors.

The positions and specific arrangement of the electrodes 104 at the different locations on the patient affects the mathematical transfer functions that are derived and/or selected. The electrode positions and arrangement on the patient are referred to herein as an electrode configuration. Two different electrode configurations may use the same electrode positions on the body (e.g., the EASI-specific locations), but with a different arrangement of the specific electrodes 104 at those positions. For example, the first electrode 104a is located at the left side chest location in a first electrode configuration, but the second electrode 104b may be located at the left side chest location in a second electrode configuration. The processor(s) 118 determine (e.g., select, derive, or calculate) different mathematical transfer functions for different electrode configurations. The mathematical transfer functions are dependent on the specific electrode configuration on the patient as well as the assumed electrode locations.

In an example, the relationship between the actual, EASI-specific, locations and the assumed electrode locations, used to determine the mathematical transfer functions, is shown in Table 1 below.

**Table 1**

| **Assumed Electrode Location** | **Actual Electrode Location** |
|---|---|
| Left Arm ("LA") | Left Side Chest ("A") |
| Right Arm ("RA") | Right Side Chest ("I") |
| Left Leg ("LL") | Upper Sternum ("S") |
| Right Leg ("RL") | Ground ("G") |
| Chest ("C") | Lower Sternum ("E") |

In this manner, Table 1 can be used to determine the relationship between the assumed electrode location and the actual electrode location by identifying the location of the assumed electrode location and identifying the location of the actual electrode location and comparing them to one another. The information in Table 1 is also shown in Figure 4. For example, Figure 4 shows that the first electrode 104a, expected, i.e., assumed, to be placed on the left arm (LA), is actually affixed to the left side chest (A) location. The second electrode 104b is actually affixed to the right side chest (I) location rather than the conventional right arm location.

In an example, the ECG system 100 may set the electrode configuration that is shown in Figure 4 as a default configuration. For example, the clinician may be instructed by the ECG system 100 or accompanying literature to arrange the electrodes 104a-e at the specific locations shown in Figure 4. In another example, the clinician has the option to select the electrode configuration on the patient. The clinician may input information indicating the selected electrode configuration to the ECG system 100, which enables the processor(s) 118 to determine the relationship between the assumed electrode locations and the actual electrode locations and derive the relevant mathematical transfer functions based on the determined relationship. The clinician may provide that information by sending a message from a remote device (e.g., a smartphone) or by inputting the information using an associated user input device 123 of the ECG system 100. The user input device 123 may be a physical keyboard, a virtual keyboard on a touch screen, or the like. Upon receiving the information indicating the selected electrode configuration, the processor(s) 118 may obtain the relevant mathematical transfer functions. The processor(s) 118 may determine the relevant mathematical transfer functions from a table or other tool that links electrode configurations to associated transfer functions. The transfer functions and/or the table or other tool may be stored and accessed from the memory 120 or another storage device.

According to the relationship between the actual electrode location of electrodes 104 and the EASI (assumed electrode) locations shown in Table 1 and Figure 4, the mathematical transfer functions in a first example include the following equations: $ES = V - 2 / 3 * aVF$ $AS = 2 / 3 * \left(aVL-aVF\right)$ $AI = 2 / 3 * \left(aVL-aVR\right)$
Where *V, aVF, aVL,* and *aVR* are four leads of the 7-lead ECG data. The one or more processors are configured to determine values of the leads V, *aVF, aVL,* and aVR from the 7-lead ECG data that is generated. The derivation of these equations 1, 2, and 3 is described herein.

In a second example using the relationship shown in Table 1 and Figure 4, the mathematical transfer functions include the following equations: $ES = V - 2 / 3 * aVF$ $AS = - III$ $AI = I$
Where *I* and *III,* along with *V* and *aVF*, are leads of the 7-lead ECG data. The one or more processors 118 are configured to determine values of the leads *V, aVF, I,* and *III* from the 7-lead ECG data that is generated. For example, the voltage value of reference vector AS is simply the inverse (e.g., negative) of the value of the lead *III,* and the voltage value of reference vector AI is simply the value of the lead *I*. The derivation of equations 4 and 5 is described herein.

After determining the mathematical transfer functions based on the electrode configuration, the processor(s) 118 input the values of the relevant leads of the 7-lead ECG data into the mathematical transfer functions to calculate the voltage values of the EASI reference vectors. In the examples above, values for the leads *V* and *aVF* are input into Equation 1 to calculate the voltage value of the reference vector ES. In the first example, the values of leads *aVL* and *aVF* are input into Equation 2 to calculate the voltage value of the reference vector AS. The values of leads *aVL* and *aVR* are input into Equation 3 to calculate the voltage value of the reference vector AI. In the second example, rather than using equations 2 and 3, the processor(s) 118 may use equations 4 and 5. For example, the voltage value of the reference vector AS is calculated as the inverse of the value of lead *III,* and the voltage value of the reference vector AI is calculated as the value of lead *I*.

After calculating the voltage values for the reference vectors, the processor(s) 118 may multiply the calculated voltage values of the reference vectors by a set of predetermined coefficients to generate the 12-lead ECG data. The 12-lead ECG data may include data for multiple precordial leads associated with the chest of the patient to enable assessment of ECG morphology changes and deflection peak time delay during LBBAP implant procedures and post-implant evaluations of pacing effectiveness.

The set of predetermined coefficients may be derived from historical data from a large pool of patients. The set of coefficients may be stored in the memory 120 or another storage device that is accessible to the processor(s) 118. In the example in which the electrodes 104 are actually located at the EASI-specific locations and the reference vectors are EASI reference vectors ES, AS, and AI, the set of coefficients are EASI coefficients, which are part of the EASI lead system. Table 2 below shows an example set of EASI coefficients for reconstructing standard 12-lead ECG data. The data in Table 2 is provided in Feild, Feldman, and Horacek, Improved EASI Coefficients: Their Derivation, Values, and Performance, J Electrocardiol. 35 Suppl: 23-33, 2002.

**Table 2**

| Lead | ES | AS | AI |
|---|---|---|---|
| I | 0.026 | -0.174 | 0.701 |
| II | -0.002 | 1.098 | -0.763 |
| III | -0.028 | 1.272 | - 1.464 |
| aVR | -0.012 | -0.462 | 0.031 |
| aVL | 0.027 | -0.723 | 1.082 |
| aVF | -0.015 | 1.185 | -1.114 |
| V1 | 0.641 | -0.391 | 0.080 |
| V2 | 1.229 | -1.050 | 1.021 |
| V3 | 0.947 | -0.539 | 0.987 |
| V4 | 0.525 | 0.004 | 0.841 |
| V5 | 0.179 | 0.278 | 0.630 |
| V6 | -0.043 | 0.431 | 0.213 |

The processor(s) 118 may multiply the voltage values of the EASI reference vectors by the corresponding coefficients in Table 2 to generate values for the leads of the 12-lead ECG data. The twelve leads of the 12-lead ECG data may include *I, II, III, aVR, aVL, aVF, V1, V2, V3, V4, V5,* and *V6.* As an example, the value of the lead *I* may be determined by multiplying the voltage value of vector ES by 0.026, multiplying the voltage value of vector AS by -0.174, and multiplying the voltage value of vector AI by 0.701, and then adding the three values. For example, the value of derived lead *I* may be equal to (0.026*ES)+(-0.174*AS)+(0.701*AI). The derived lead *II* is determined in a similar way as lead *I,* except the corresponding coefficients for lead *II* are -0.002, 1.098, and -0.763. The value of derived lead II may be equal to (-0.002*ES)+(1.098*AS)+(-0.763*AI). The values of the other ten derived leads are calculated in a similar way as derived leads *I* and *II.* The values of the twelve derived leads represent the 12-lead ECG data.

In an example, the set of predetermined coefficients shown in Table 2 may represent a first set of multiple different sets of predetermined coefficients that are accessible to the one or more processors 118. The multiple sets of coefficients may be stored in a database, such as the memory 120 or a remote data storage device. The one or more processors 118 may select the first set of coefficients, from the multiple sets of coefficients in the database, such as the memory 120, based on a target implant location of a lead of an IMD 124 within the patient and/or a condition of the patient. For example, the first set of coefficients may be associated in the database, such as the memory 120, with LBBAP, and the processor(s) 118 may select the first set in response to receiving a signal indicating that the user is interested in implanting a lead of an IMD 124 into the left bundle branch for LBBAP. In response to receiving a signal indicating that the user is interested in implanting the lead of the IMD 124 in the right atrium, for example, the processor(s) 118 may select a different, second set of predetermined coefficients from the database, such as the memory 120, to use to generate the converted ECG data. In an example, the processor(s) 118 may update and/or modify the predetermined coefficients in the set(s) over time based on historical data. As an example, machine learning algorithms may be used to refine the predetermined coefficients using data collected during actual procedures.

After determining the 12-lead ECG data, the processor(s) 118 may perform one or more operations based on the 12-lead ECG data. For example, the processor(s) 118 may display the 12-lead ECG data on a display device (not shown in Figure 3) that is communicatively connected to or included in the programmer device 102. The processor(s) 118 may control the display device to display a graphical representation (e.g., indicia) of the 12-lead ECG data for viewing by a clinician. The clinician can refer to the displayed 12-lead ECG data when implanting an IMD 124 and/or evaluating the effectiveness of an implanted IMD 124.

In an example, the programmer device 102 may use the 12-lead ECG data to select a pacing site of a transvenous lead of the IMD 124 within the patient. For example, the lead of the IMD 124 may be implanted into the heart, and more specifically may penetrate the intraventricular septal wall to reach the LBB. During the implant procedure to implant the lead in the heart, the ECG system 100 may be activated to monitor the patient by generating 12-lead ECG data. For example, the clinician may move the distal end of the implantable lead to various pacing (or implant) sites within the septal wall in an effort to select a site that is preferred over one or more other sites. The sites may differ with respect to the location along the septal wall (e.g., in vertical and lateral directions) and the depth of penetration into the thickness of the septal wall (e.g., in depth direction).

At each site, the IMD 124 may be controlled to generate one or more pacing pulses that are emitted into the septal wall by the lead. The ECG system 100 may monitor the electrical responses of the patient's conductive system to the pacing pulses. The processor(s) 118 may generate the 12-lead ECG data indicating the electrical responses to the pacing pulses. The processor(s) 118 in an example may compare the 12-lead ECG data associated with the different pacing sites. The processor(s) 118 may select one of the sets of 12-lead ECG data that reflects that the pacing pulse(s) more efficiently captured the conductive system of the heart than the pacing pulse(s) emitted by the lead at one or more other pacing sites. For example, the processor(s) 118 may compare the values of the V1 and V6 leads of the 12-lead ECG data in response to the pacing pulse(s) emitted by the lead at each of the different pacing sites. The processor(s) 118 may select the pacing site for permanent lead implant based on the comparison of the 12-lead ECG data. For example, the processor(s) 118 may select the pacing site that is associated with the ECG data that has the greatest amplitude, relative to the amplitudes of the ECG data associated with the other tested pacing sites. After selecting the pacing site, the processor(s) 118 may generate a message to notify the clinician of the selected pacing site. The processors(s) 118 may control a display device to display the message to the clinician.

In another example, the programmer device 102 is configured to select pacing parameters of the IMD 124 based on the 12-lead ECG data. The pacing parameters may be selected for controlling stimulation therapy administered by the transvenous lead of the IMD 124 to the patient. In an example application, the processor(s) 118 may select the pacing parameters during the implant procedure described above or at a later time, post-implant, such as during a follow-up appointment with the clinician to evaluate the effectiveness of the stimulation therapy provided by the IMD 124. To select the pacing parameters, the processor(s) 118 may monitor the 12-lead ECG data that indicate the electrical responses of the patient's conductive system to the IMD 124 performing a test. The test may involve the IMD 124 generating a sequence of pacing pulses having different pacing parameters over time. The pacing parameters may refer to the amplitude or intensity, the phase, the vector, the duration, the frequency, and/or the like of the pacing pulses.

The processor(s) 118 may compare the 12-lead ECG data associated with the different pacing parameters. The processor(s) 118 may select one of the sets of 12-lead ECG data that reflects that the pacing pulse(s) more efficiently captured the conductive system of the heart than the pacing pulse(s) emitted by the lead via different pacing parameters. For example, the processor(s) 118 may compare the values of the V1 and V6 leads of the 12-lead ECG data in response to the pacing pulse(s) emitted by the lead via each of the different pacing parameters. The processor(s) 118 may select the pacing parameters for the IMD based on the comparison of the 12-lead ECG data. For example, the processor(s) 118 may select the pacing parameters that are associated with the ECG data that has the greatest sensed amplitude in the ECG data, relative to the sensed amplitudes of the ECG data associated with the other tested pacing parameters. After selecting the pacing parameters, the processor(s) 118 may generate a control signal or message that is communicated to the IMD 124. The control signal or message may instruct the IMD 124 to implement the selected pacing parameters for future pacing therapy. Furthermore, the processor(s) 118 may generate a message to notify the clinician of the selected pacing parameters, which may represent a modification of previous pacing parameters implemented by the IMD 124. The processors(s) 118 may control a display device to display the message to the clinician.

The mathematical transfer functions indicated by Equations 1, 2, and 3 above may be derived based on the following ECG equations: $Vw = 1 / 3 * \left(RA+LA+LL\right)$ $aVR = RA - \frac{1}{2} * \left(LA+LL\right)$ $aVL = LA - \frac{1}{2} * \left(RA+LL\right)$ $aVF = LL - \frac{1}{2} * \left(RA+LA\right)$ $3 / 2 * V = 3 / 2 * \left(C-Vw\right)$

The variable Vw represents the voltage at the Wilson's central terminal (WCT). The variables RA, LA, LL, and C represent the electrodes 104 designed to be affixed to the right arm, the left arm, the left leg, and the chest, respectively. In the electrode configuration shown in Figure 4, the RA variable represents the second electrode 104b that is affixed to the right side chest (I) location, the LA variable represents the first electrode 104a that is affixed to the left side chest (A) location, the LL variable represents the third electrode 104c that is affixed to the upper sternum (S) location, and the C variable represents the fifth electrode 104e that is affixed to the lower sternum (E) location. The right leg or RL electrode is not present in these equations because it is used as a reference electrode. Combining Equation A with each of Equations B through D yields the following equations: $aVR = 3 / 2 * \left(RA-Vw\right)$ $aVL = 3 / 2 * \left(LA-Vw\right)$ $aVF = 3 / 2 * \left(LL-Vw\right)$

Subtracting equation H from E yields: $3 / 2 * V - aVF = 3 / 2 * \left(C-LL\right) = 3 / 2 * ES$

Subtracting equation H from G yields: $aVL - aVF = 3 / 2 * \left(LA-LL\right) = 3 / 2 * AS$

Subtracting equation F from G yields: $aVL - aVR = 3 / 2 * \left(LA-RA\right) = 3 / 2 * AI$

Equation I can be rewritten to solve for ES, producing Equation 1 as follows: $ES = 2 / 3 * \left(3/2*V-aVF\right)$ $ES = V - 2 / 3 * aVF$

Equation J can be rewritten to solve for AS, producing Equation 2: $AS = 2 / 3 * \left(aVL-aVF\right)$

Equation K can be rewritten to solve for AI, producing Equation 3: $AI = 2 / 3 * \left(aVL-aVR\right)$

In the second example described above, the mathematical transfer functions indicated by Equations 4 and 5 to calculate reference vectors AS and AI are derived based on the following ECG equations and logic. The reference vector ES is calculated according to Equation 1, which is derived above. For example, the ECG system 100 may generate lead *III* of the 7-lead ECG data as the bipolar vector from electrode LL to electrode LA (e.g., the third electrode 104c to the first electrode 104a). As shown in Figure 4, the vector AS is from the A location to the S location, which is from the LA electrode 104a to the LL electrode 104c. Based on this logic, the AS vector is simply the inverse or negative of the lead *III.* Thus, Equation 4 is AS = -III.

The ECG system 100 may generate lead I of the 7-lead ECG data as the bipolar vector from electrode LA to electrode RA (e.g., the first electrode 104a to the second electrode 104b). As shown in Figure 4, the vector AI is from the A location to the I location, which is from the LA electrode 104a to the RA electrode 104b. Based on this logic, the AI vector is equivalent to the lead I. Thus, Equation 5 is AI = I.

In another example, the mathematical transfer functions for the EASI reference vectors ES, AS, and AI may be determined by deriving a new set of signals. This third example method may be used instead of the Equations 1 through 3 or Equations 1, 4, and 5. The ECG system 100 may generate lead *II* of the 7-lead ECG data as the bipolar vector from the LL electrode 104c to the RA electrode 104b. This third method assumes voltage at the LL electrode 104c is always zero. Using this assumption, the processor(s) 118 calculate LA and RA voltage using lead *II* and *III* of the 7-lead ECG data by the following equations: $RA = LL - II = 0 - II = - II$ $LA = LL - III = 0 - III = - III$

Note that the calculated RA and LA voltage here is not the unipolar signal with RL as the reference. Instead, the voltages use the LL unipolar signal as the new reference. To distinguish these voltage values from the unipolar signals, the terms RA', LA', and LL' are used: $RA ′ = RA - LL$ $LA ′ = LA - LL$ $LL ′ = LL - LL = 0$

Next, the derivation calculates a WCT' by the following: $WCT ′ = \left(LA′+RA′+LL′\right) / 3 = \left(LA+RA+LL\right) / 3 - 3 * LL / 3 = WCT - LL$

Next, the derivation calculates the term C' by the following: $C ′ = C - LL = \left(V+WCT\right) - LL = V + \left(WCT-LL\right) = V + WCT ′$

In this way, there is a new set of electrode signals identified as LA', RA', LL', and C'. This new set of signals is equivalent to the unipolar voltage at each of the LA, RA, LL, C electrodes minus the unipolar voltage at the LL electrode 104c.

The EASI reference vectors can be solved for, as shown in the following equations: $ES = C - LL = C ′ - LL ′ = C ′ = V - \left(II+III\right) / 3$ $AS = LA - LL = LA ′ - LL ′ = LA ′ = - III$ $AI = LA - RA = LA ′ - RA ′ = II - III$

The equations O, P, and Q can be used as the mathematical transfer functions in this third example. For example, the processor(s) 118 may calculate the voltage value of the reference vector ES as the value of the signal C'. The voltage value of the reference vector AS can be calculated as the value of the signal LA'. The voltage value of the reference vector AI can be calculated as the value of the signal LA' minus the value of the signal RA'.

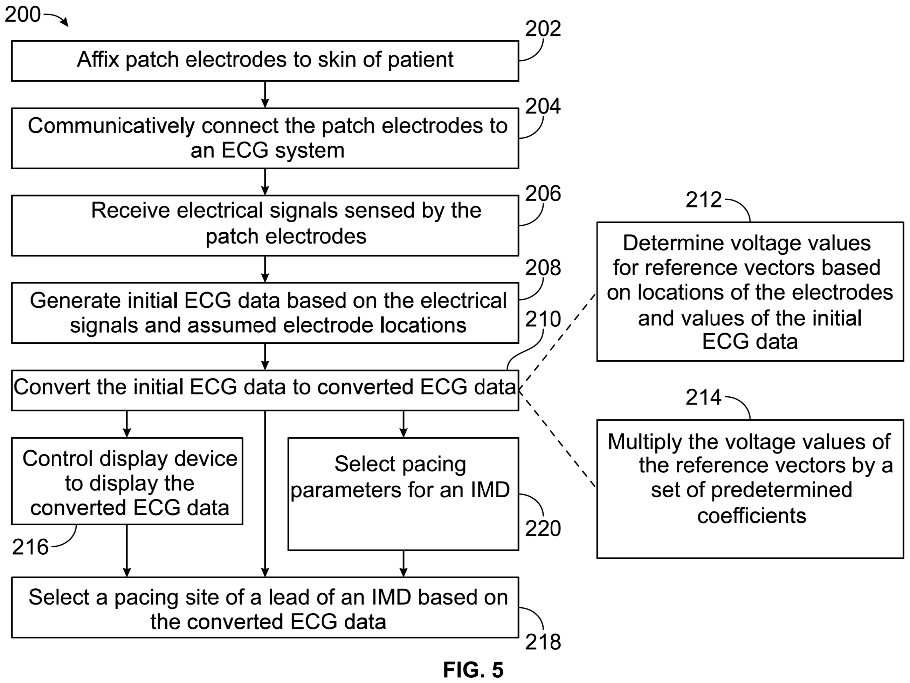

Figure 5 is a flow chart of a method 200 for generating, for instance, 12-lead ECG data using a reduced number of electrodes according to an embodiment. The method 200 may use the ECG system 100 shown in Figures 3 and 4. In different embodiments, the method may include different steps not shown in Figure 5, may omit one or more of the steps shown in Figure 5, and/or may have a different order of the steps than the order shown in Figure 5.

At step 202, patch electrodes 104 of the ECG system 100 are affixed to the skin of a patient at specific actual electrode locations. In an embodiment, the actual electrode locations of the patch electrodes 104 may be exclusively on the patient's torso. In a particular embodiment, the locations on the torso may be at EASI-specific locations A, E, S, I, and G, as shown in Figure 4. The ECG system 100 may, in an embodiment, use five patch electrodes 104. The patch electrodes 104 may, for instance, be affixed using medical grade adhesive.

At step 204, the patch electrodes 104 are communicatively connected to the ECG system 100. For example, the patch electrodes 104 may be electrically connected to different corresponding wires 106 of an electrical cable 108. The method 200 may include electrically connecting the electrical cable 108 to a port 112 of a computing device 102 of the ECG system 100.

The following steps may be performed by the computing device 102, and more specifically by one or more processors 118 (e.g., hardware circuitry) of the computing device 102. At step 206, the computing device 102 of the ECG system 100 obtains electrical signals that are sensed by the patch electrodes 104 affixed to the skin of the patient at the actual electrode locations. The computing device 102 may receive the electrical signals contemporaneously with the patch electrodes 104 sensing the electrical signals. Alternatively, the electrical signals sensed by the patch electrodes 104 may be stored in the memory 120 or another data storage device, and the processor(s) 118 of the computing device 102 may obtain the electrical signals by accessing the electrical signals from the memory 120 or other data storage device. At step 208, the processor(s) 118 of the computing device 102 generate initial (e.g., 7-lead) ECG data based on the electrical signals sensed by the patch electrodes 104 while affixed at the actual electrode locations on the patient. The initial ECG data is based on the sensed electrical signals according to assumed electrode locations of the patch electrodes 104 on the patient. The assumed electrode locations differ from the actual electrode locations of the patch electrodes 104 while the patch electrodes 104 sensed the electrical signals. For example, the initial ECG data may include voltage potentials (e.g., values) along one or more particular bipolar vectors that are not actually at the assumed locations on the patient.

At step 210, the processor(s) 118 convert the initial ECG data to converted (e.g., 12-lead) ECG data. This operation may be completed, at least in part, by sub-steps 212 and 214. As an initial sub-step, a relationship between the actual electrode locations of the patch electrodes 104 and assumed electrode locations is determined. For example, particular patch electrodes 104 connected via different wires to the computing device 102 may be expected to be affixed at different assumed electrode locations on the patient. The actual electrode locations do not match the assumed electrode locations. The correlation between the assumed and actual location of each patch electrode 104 is determined by the processor(s) 118. For example, the processor(s) 118 may access a data file from the memory 120 that provides the correlation between the assumed and actual locations of the patch electrodes 104. The patch electrodes 104 may have unique identifiers, such as ID numbers or serial numbers, disposed on the patch electrodes 104. During step 202, the processor(s) 118 may instruct the user where to place each of the specific patch electrodes 104 so that the actual electrode locations conform to a known relationship between the assumed and actual electrode locations. For example, the processor(s) 118 may display instructions on an integrated display device that instruct the user to place electrode 104a at the left torso (e.g., the "A" location in Figure 4) instead of the left arm, to place electrode 104c at the upper chest ("S" location in Figure 4) instead of the left leg, and so on. Thus, the relationship may be pre-selected and used to instruct the user where to place the particular patch electrodes 104 on the patient. In another example, the processor(s) 118 may determine the relationship between the actual and assumed electrode locations by the user taking a photograph of the patch electrodes 104 affixed to the patient and uploading the photograph to the computing device 102 for analysis.

At step 212, the processor(s) 118 determine voltage values for reference vectors based on the relationship between the assumed and actual locations of the patch electrodes 104 on the patient and values of the initial ECG data. In an example, the reference vectors are the EASI vectors AS, ES, and AI. In an embodiment, the processor(s) 118 may determine the voltage values for the reference vectors by first providing/determining, such as selecting or deriving, mathematical transfer functions based on the relationship between the assumed and actual locations of the patch electrodes 104 on the patient. After determining the mathematical transfer functions, the processor(s) 118 may input values of at least some leads of the initial (e.g., 7-lead) ECG data into the mathematical transfer functions. The output of the mathematical transfer functions may be the voltage values for the reference vectors. At step 214, the processor(s) 118 multiply the voltage values for the reference vectors by a set of predetermined coefficients to generate the converted (e.g., 12-lead) ECG data.

At optional step 216, a display device is controlled by the processor(s) 118 to display the converted ECG data or a subset of the converted ECG data for viewing by a clinician. For example, the subset of converted ECG data for display can be chest lead V1 and V6, plus a few limb lead such as I, II, III, aVF.

At optional step 218, the processor(s) 118 may select, based on the converted ECG data, a pacing site of an implantable cardiac lead of an IMD 124 implanted within the patient.

At optional step 220, the processor(s) 118 may select, based on the converted ECG data, pacing parameters of the IMD 124 implanted within the patient for controlling stimulation therapy administered by the IMD 124 to the patient. As indicated by the flow chart progression, the steps 216, 218, and 220 may be independent from one another.

Further, the disclosure comprises examples according to the following embodiments:
An aspect of the present technology relates to an electrocardiogram (ECG) system 100 comprising a computing device 102 configured to be communicatively connected to patch electrodes 104 that are affixed to skin of a patient. The computing device 102 includes one or more processors 108 configured to obtain electrical signals sensed by the patch electrodes 104 while the patch electrodes are located on the patient at actual electrode locations. The one or more processors 108 are also configured to generate initial ECG data based on the electrical signals from the patch electrodes 104 according to assumed electrode locations on the patient that differ from the actual electrode locations. The one or more processors 108 are further configured to convert the initial ECG data to converted ECG data via determining a relationship between the assumed electrode locations and the actual electrode locations, determining voltage values for reference vectors based on the initial ECG data and the relationship that is determined, and multiplying the voltage values for the reference vectors by a set of predetermined coefficients.

In an embodiment, the initial ECG data is 7-lead ECG data and the converted ECG data is 12-lead ECG data.

In an embodiment, the electrical signals are sensed by five patch electrodes 104.

In an embodiment, the actual electrode locations of the patch electrodes 104 that sense the electrical signals are all on a torso of the patient, and one or more of the assumed electrode locations are on a limb of the patient.

In an embodiment, the assumed electrode locations include a left arm of the patient, a left leg of the patient, a right arm of the patient, a right leg of the patient, and a chest of the patient.

In an embodiment, the patch electrodes 104 are communicatively connected to the one or more processors 108 via wires 106 of an electrical cable 116.

In an embodiment, the one or more processors 108 are configured to determine the voltage values for the reference vectors by inputting the values of the initial ECG data into mathematical transfer functions that output the voltage values for the reference vectors. In this embodiment, the one or more processors 108 are configured to determine the mathematical transfer functions based on the relationship between the assumed electrode locations and the actual electrode locations.

In an embodiment, the reference vectors are EASI reference vectors including an AS vector, an ES vector, and an AI vector, and the actual electrode locations of the patch electrodes 104 on the patient are EASI-specific locations.

In an embodiment, the EASI-specific locations of the patch electrodes 104 include a left side chest location, a right side chest location, an upper sternum location, a lower sternum location, and a ground location, wherein the ground location is below a sixth rib of a rib cage of the patient.

In an embodiment, the AS vector has a diagonal orientation from a left side chest location to an upper sternum location, the AI vector has a horizontal orientation from the left side chest location to a right side chest location, and the ES vector has a vertical orientation from a lower sternum location to the upper sternum location.

In an embodiment, the relationship between the assumed electrode locations and the EASI-specific locations of the patch electrodes (e.g., actual electrode locations) on the patient is:
(i) a left arm ("LA") assumed electrode location corresponds to a left side chest ("A") EASI-specific location; (ii) a right arm ("RA") assumed electrode location corresponds to a right side chest ("I") EASI-specific location; (iii) a left leg ("LL") assumed electrode location corresponds to an upper sternum ("S") EASI-specific location; (iv) a right leg ("RL") assumed electrode location corresponds to a ground ("G") EASI-specific location; and a chest ("C") assumed electrode location corresponds to a lower sternum ("E") EASI-specific location.

In an embodiment, the one or more processors 117 are configured to determine the voltage values for the reference vectors by inputting values of the initial ECG data into mathematical transfer functions that output the voltage values for the reference vectors. In a particular embodiment, the mathematical transfer functions include: $ES = V - 2 / 3 * aVF$ $AS = 2 / 3 * \left(aVL-aVF\right)$ $AI = 2 / 3 * \left(aVL-aVR\right)$ wherein *V, aVF, aVL,* and *aVR* are four leads of the initial ECG data, and the one or more processors 118 are configured to determine values of the leads *V, aVF,* aVL, and aVR from the initial ECG data that is generated.

In an embodiment, the one or more processors 118 are configured to determine the voltage values for the reference vectors by inputting values of the initial ECG data into mathematical transfer functions that output the voltage values for the reference vectors. In a particular embodiment, the mathematical transfer functions include: $ES = V - 2 / 3 * aVF$ $AS = - III$ $AI = I$ wherein *V, aVF, III,* and *I* are four leads of the initial ECG data, and the one or more processors 118 are configured to determine values of the leads *V, aVF, III*, and *I* from the initial ECG data that is generated.

In an embodiment, the ECG system 100 further comprises a display device communicatively connected to the one or more processors 118 of the computing device 102. In this embodiment, the one or more processors 118 are configured to control the display device to display a graphical representation of the converted ECG data for viewing by a clinician.

In an embodiment, the computing device 102 is communicatively connected to an IMD 124 that is configured to be implanted within the patient. In this embodiment, the computing device 102 is configured to select a pacing site of an implantable cardiac lead of the IMD 124 based on the converted ECG data.

In an embodiment, the computing device 102 is communicatively connected to an IMD 124 that is configured to be implanted within the patient. In this embodiment, the one or more processors 118 of the computing device 102 are configured to select pacing parameters of the IMD 124 based on the converted ECG data. In this embodiment, the one or more processors 118 are configured to select the pacing parameters for controlling stimulation therapy administered by the IMD 124 to the patient.

In an embodiment, the one or more processors 118 are communicatively connected to a user input device 123. In this embodiment, the one or more processors 118 are configured to toggle between a data conversion mode of operation and a standard mode of operation based on a signal received from the user input device 123 indicative of a user selection. In this embodiment, the one or more processors 118 are configured to convert the initial ECG data to the converted ECG data in the data conversion mode and are configured to not convert the initial ECG data in the standard mode.

In an embodiment, the set of predetermined coefficients represents a first set of coefficients of multiple sets of coefficients stored in a memory device, such as memory 120. In this embodiment, the one or more processors 118 are configured to select the first set of coefficients, from the multiple sets of coefficients in the memory device, based on at least one of a target implant location of a lead of an IMD 124 within the patient or a condition of the patient.

Another aspect of the present technology relates to a method comprising obtaining, via one or more processors 118 of a computing device 102, electrical signals sensed by patch electrodes 104. The patch electrodes 104 sense the electrical signals while affixed to skin of a patient at actual electrode locations. The method also comprises generating initial ECG data based on the electrical signals received from the patch electrodes 104 according to assumed electrode locations on the patient that differ from the actual electrode locations. The method further comprises converting the initial ECG data to converted ECG data by determining a relationship between the assumed electrode locations and the actual electrode locations, determining voltage values for reference vectors based on the initial ECG data and the relationship that is determined, and multiplying the voltage values for the reference vectors by a set of predetermined coefficients.

In an embodiment, the method further comprises controlling a display device to display a graphical representation of the converted ECG data for viewing by a clinician.

In an embodiment, the method further comprises receiving a user selection signal via a user input device 123 communicatively connected to the one or more processors 118. In this embodiment, the method also comprises toggling between a data conversion mode of operation and a standard mode of operation based on the user selection signal. In this embodiment, converting the initial ECG data to the converted ECG data occurs in the data conversion mode but not in the standard mode.

In an embodiment, the reference vectors are EASI reference vectors including an AS vector, an ES vector, and an AI vector, and the actual electrode locations of the patch electrodes 104 on the patient are EASI-specific locations.

In an embodiment, determining the voltage values for the reference vectors comprises inputting values of the initial ECG data into mathematical transfer functions that output the voltage values for the reference vectors. In this embodiment, the method further comprises determining the mathematical transfer functions based on the relationship between the assumed electrode locations and the actual electrode locations.

In an embodiment, the method further comprises electrically connecting the patch electrodes 104 to the computing device 102 via an electrical cable 116.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

The terms "about" and "approximately" immediately preceding a stated numerical value, as used herein, indicate that the actual value can be +/- a designated threshold of the stated numerical value. The designated threshold may be 5%, 10% or the like of the stated numerical value.

In general, the various features and examples described herein can be combined unless the combination of a first feature with a second feature would frustrate the function of one of the features or render one of the features useless.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the inventive subject matter without departing from its scope. While the dimensions and types of materials described herein are intended to define the parameters of the inventive subject matter, they are by no means limiting and are exemplary embodiments. Many other embodiments will be apparent to one of ordinary skill in the art upon reviewing the above description. The scope of the inventive subject matter should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

## Claims

1. An electrocardiogram, ECG, system (100) comprising:
a computing device (102) configured to be communicatively connected to patch electrodes (104) that are affixed to skin of a patient, the computing device (102) including one or more processors (118) configured to:
obtain electrical signals sensed by the patch electrodes (104) while affixed on the patient at actual electrode locations;
generate initial ECG data based on the electrical signals from the patch electrodes (104) according to assumed electrode locations on the patient that differ from the actual electrode locations; and
convert the initial ECG data to converted ECG data via:
determining a relationship between the assumed electrode locations and the actual electrode locations;
determining voltage values for reference vectors based on the initial ECG data and the determined relationship; and
generating the converted ECG data by multiplying the voltage values for the reference vectors by a set of predetermined coefficients.

2. The ECG system of claim 1, wherein the initial ECG data is 7-lead ECG data and the converted ECG data is 12-lead ECG data.

3. The ECG system of claim 1 or 2, wherein the electrical signals are sensed by five patch electrodes (104).

4. The ECG system of any one of claims 1 to 3, wherein the actual electrode locations of the patch electrodes (104) that sense the electrical signals are all on a torso of the patient, and one or more of the assumed electrode locations are on a limb of the patient, preferably wherein the assumed electrode locations include a left arm of the patient, a left leg of the patient, a right arm of the patient, a right leg of the patient, and a chest of the patient.

5. The ECG system of any one of claims 1 to 4, wherein the one or more processors (118) are configured to:
determine the voltage values for the reference vectors by inputting values of the initial ECG data into mathematical transfer functions that output the voltage values for the reference vectors; and
determine the mathematical transfer functions based on the relationship between the assumed electrode locations and the actual electrode locations.

6. The ECG system of any one of claims 1 to 5, wherein the reference vectors are EASI reference vectors including an AS vector, an ES vector, and an AI vector, and the actual electrode locations of the patch electrodes on the patient are EASI-specific locations, preferably wherein the EASI-specific locations of the patch electrodes (104) include a left side chest location, a right side chest location, an upper sternum location, a lower sternum location, and a ground location, wherein the ground location is below a sixth rib of a rib cage of the patient; and/or wherein the AS vector has a diagonal orientation from a left side chest location to an upper sternum location, the AI vector has a horizontal orientation from the left side chest location to a right side chest location, and the ES vector has a vertical orientation from a lower sternum location to the upper sternum location.

7. The ECG system of claim 6, wherein the relationship between the assumed electrode locations and the EASI-specific locations of the patch electrodes (104) on the patient is:
(i) a left arm ("LA") assumed electrode location corresponds to a left side chest ("A") EASI-specific location; (ii) a right arm ("RA") assumed electrode location corresponds to a right side chest ("I") EASI-specific location; (iii) a left leg ("LL") assumed electrode location corresponds to an upper sternum ("S") EASI-specific location; (iv) a right leg ("RL") assumed electrode location corresponds to a ground ("G") EASI-specific location; and a chest ("C") assumed electrode location corresponds to a lower sternum ("E") EASI-specific location.

8. The ECG system of claim 7, wherein the one or more processors (118) are configured to determine the voltage values for the reference vectors by inputting values of the initial ECG data into mathematical transfer functions that output the voltage values for the reference vectors, wherein
the mathematical transfer functions include: $ES = V - 2 / 3 * aVF$ $AS = 2 / 3 * \left(aVL-aVF\right)$ $AI = 2 / 3 * \left(aVL-aVR\right)$
wherein *V, aVF, aVL,* and aVR are four leads of the initial ECG data, and the one or more processors (118) are configured to determine values of the leads *V*, *aVF, aVL,* and aVR from the initial ECG data that is generated; or
the mathematical transfer functions include: $ES = V - 2 / 3 * aVF$ $AS = - III$ $AI = I$
wherein *V, aVF, III,* and *I* are four leads of the initial ECG data, and the one or more processors are configured to determine values of the leads *V, aVF, III,* and *I* from the initial ECG data that is generated.

9. The ECG system of any one of claims 1 to 8, wherein the computing device (102) is communicatively connected to an implantable medical device, IMD, (124) implanted within the patient, and the one or more processors (118) of the computing device (102) are configured to select a pacing site of an implantable cardiac lead of the IMD (!02) based on the converted ECG data.

10. The ECG system of any one of claims 1 to 9, wherein the computing device (!02) is communicatively connected to an implantable medical device, IMD, (124) implanted within the patient, and the one or more processors (118) of the computing device (102) are configured to select pacing parameters for controlling stimulation therapy administered by the IMD (124) to the patient based on the converted ECG data.

11. The ECG system of any one of claims 1 to 10, wherein the one or more processors (118) are communicatively connected to a user input device (123), and the one or more processors (118) are configured to:
toggle between a data conversion mode of operation and a standard mode of operation based on a signal received from the user input device (118) indicative of a user selection;
convert the initial ECG data to the converted ECG data in the data conversion mode: and
not convert the initial ECG data in the standard mode.

12. The ECG system of any one of claims 1 to 11, wherein the set of predetermined coefficients represents a first set of coefficients of multiple sets of coefficients stored in a memory (120), wherein the one or more processors (118) are configured to select the first set of coefficients, from the multiple sets of coefficients in the memory (120), based on a target implant location of an IMD (124) within the patient.

13. A method comprising:
obtaining, via one or more processors (118) of a computing device (102), electrical signals sensed by patch electrodes (104), wherein the patch electrodes (104) sense the electrical signals while affixed to skin of a patient at actual electrode locations;
generating initial ECG data based on the electrical signals from the patch electrodes (!04) according to assumed electrode locations on the patient that differ from the actual electrode locations; and
converting the initial ECG data to converted ECG data by:
determining a relationship between the assumed electrode locations and the actual electrode locations;
determining voltage values for reference vectors based on the initial ECG data and the relationship that is determined; and
generating the converted ECG data by multiplying the voltage values for the reference vectors by a set of predetermined coefficients.

14. The method of claim 13, further comprising:
receiving a user selection signal via a user input device (123) communicatively connected to the one or more processors (118); and
toggling between a data conversion mode of operation and a standard mode of operation based on the user selection signal, wherein converting the initial ECG data to the converted ECG data occurs in the data conversion mode but not in the standard mode.

15. The method of claim 13 or 14, wherein determining the voltage values for the reference vectors comprises inputting values of the initial ECG data into mathematical transfer functions that output the voltage values for the reference vectors, and the method further comprises:
determining the mathematical transfer functions based on the relationship between the assumed electrode locations and the actual electrode locations.
